# EUROPÄISCHE PATENTSCHRIFT

(11) **EP 0 919 532 B1**
(45) Veröffentlichungstag und Bekanntmachung des Hinweises auf die Patenterteilung: **16.01.2002**
(21) Anmeldenummer: 98121947.0
(22) Anmeldetag: 19.11.1998
(51) Int. Cl.: C07C 45/72, C07C 49/203, C07C 29/17, C07C 29/145, C07C 31/125

(54) **Verfahren zur Herstellung von gesättigten Alkoholen**
Process for the preparation of saturated alcohols
Procédé pour la préparation d'alcools saturés

(30) Priorität: 29.11.1997 DE 19753157
(43) Veröffentlichungstag der Anmeldung: 02.06.1999
(73) Patentinhaber: Celanese Chemicals Europe GmbH, 60439 Frankfurt am Main (DE)
(72) Erfinder: Springer, Helmut, 46539 Dinslaken (DE)

(56) Entgegenhaltungen:
- GB-A- 446 026
- US-A- 2 088 017
- S.G. POWELL, ET AL.: "The condensation of isobutyraldehyde with aliphatic ketones" JOURNAL OF THE AMERICAN CHEMICAL SOCIETY, Bd. 66, Nr. 3, 9. März 1944, Seiten 372-376, XP002095352 Washington, DC, US

## Beschreibung

Die vorliegende Erfindung betrifft ein Verfahren zur Herstellung von gesättigten Alkoholen umfassend eine Aldolkondensation von am β-C-Atom verzweigten Alkylmethylketonen mit 6 - 8 Kohlenstoffatomen und am α-C-Atom verzweigten Aldehyden mit 4 - 15 Kohlenstoffatomen unter Bildung von α,β-ungesättigten Ketonen und eine nachfolgende Hydrierung der α,β-ungesättigten Ketone zu den ensprechenden gesättigten Alkoholen.

Als Aldoladdition bezeichnet man die durch Basen oder Säuren katalysierte Addition aktivierter Methylengruppen an die Carbonylgruppen von Aldehyden oder Ketonen unter Bildung von β-Hydroxycarbonylverbindungen. Hierbei stellt sich nach einer gewissen Reaktionszeit ein Gleichgewicht zwischen den β-Hydroxycarbonylverbindungen und den nicht umgesetzten Ausgangsstoffen ein. Schließt sich der Aldoladdition eine Wasserabspaltung an, die leicht eintritt und die Regel bei der Verwendung saurer Katalysatoren ist, so spricht man insgesamt von einer Aldolkondensation. Bei den Produkten der Aldolkondensation handelt es sich um α,β-ungesättigte Carbonylverbindungen.

Bei der Aldoladdition zwischen Aldehyden und Ketonen fungieren die Ketone infolge ihrer geringeren Carbonylaktivität immer als Methylenkomponente. Die Kondensation der Keto-Gruppe mit der α-Methylen-Gruppe eines Aldehyds ist nur in wenigen Fällen über besondere Zwischenstufen möglich. Man erhält daher üblicherweise zunächst β-Hydroxy-Ketone, deren Keto-Gruppe aus dem eingesetzten Keton stammt. Durch die anschließende Wasserabspaltung werden aus den β-Hydroxy-Ketonen die entsprechenden α,β-ungesättigten Ketone gebildet.

Bei der Reaktion zwischen Aldehyden und Ketonen erhält man nur ein einziges α,β-ungesättigtes Keton als Reaktionsprodukt, wenn gesättigte, aliphatische Aldehyde ohne α-ständiges Wasserstoffatom eingesetzt werden. Die Selbstkondensation des Ketons ist dabei nur in minimalem Ausmaß zu beobachten. Im Fall der Verwendung von Aldehyden mit α-ständigem Wasserstoffatom muß dagegen auch mit der Selbstkondensation der Aldehyde als unerwünschte Nebenreaktion gerechnet werden.

Während bei Verwendung symmetrischer Ketone immer nur die Bildung eines β-Hydroxy-Ketons in der Aldoladdition möglich ist, ergibt sich bei Verwendung von unsymmetrischen Ketonen, bei denen die α-ständigen Wasserstoffatome nicht gleichwertig sind, die Möglichkeit zur Bildung zweier strukturell verschiedener β-Hydroxy-Ketone gemäß dem folgenden Formelschema I, in dem R₁ nicht für Wasserstoff steht,

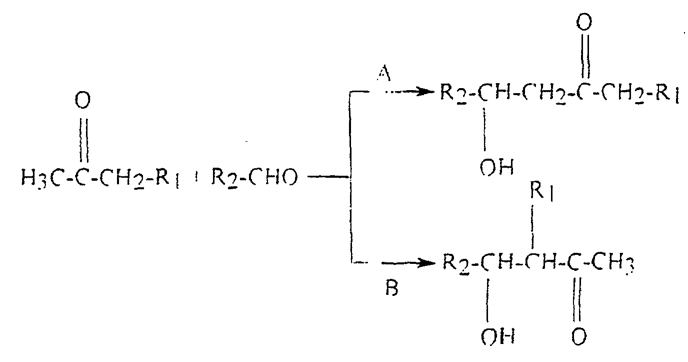

Über den Verlauf solcher Aldolkondensationen mit unsymmetrischen Ketonen lassen sich Regeln aufstellen. Hierbei ist zunächst zwischen der alkalikatalysierten und der säurekatalysierten Aldolkondensation zu unterscheiden. Für den Ablauf der Reaktion sind sowohl die CH-Acidität als auch sterische Faktoren maßgebend.

In der alkalikatalysierten Aldolkondensation erfolgt bei Verwendung unverzweigter Aldehyde und α-verzweigter Ketone die Abstrahierung des Protons vom α-C-Atom des Ketons, das die meisten Substituenten trägt (Fall A). Bei Verwendung von am α-C-Atom verzweigten Aldehyden erfolgt die Abstrahierung des Protons dagegen bevorzugt von dem α-C-Atom des Ketons, welches die wenigsten Substituenten trägt (Fall B). Im Fall A wird somit in der Aldoladdition ein β-Hydroxy-Keton gemäß Gleichung IA gebildet, während im Fall B ein Reaktionsverlauf gemäß Gleichung IB zu erwarten ist.

Ausnahmen von der obigen Regel ergeben sich, wenn das Keton in β-Stellung verzweigt ist. Infolge des zusätzlich auftretenden, sterischen Effektes erfolgt die Kondensation hier unabhängig von der Aldehyd-Struktur immer entsprechend der Gleichung IA.

Im Gegensatz zur alkalischen Katalyse erhält man bei der Aldolkondensation unter Verwendung saurer Katalysatoren in jedem Fall, d.h. unabhängig von der Verzweigungsart der Aldehyde und Ketone, einen Reaktionsverlauf gemäß Gleichung IB.

Unerwünschte Nebenreaktionen der Keton- und Aldehyd-Edukte, die mit der Aldolisierung konkurrieren, können beispielsweise die Cannizzarro-Reaktion sowie die Claisen-Tischtschenko-Reaktion sein. Üblicherweise unterliegen nur aromatische und nicht aldolisierbare aliphatische Aldehyde, d.h. Aldehyde ohne α-ständiges Wasserstoff-Atom, der Cannizzarro-Reaktion und disproportionieren in Gegenwart starker Alkalien unter Bildung äquimolarer Mengen Alkohol und Carbonsäure. Bei aldolisierbaren Aldehyden erfolgt dagegen in der Regel ausschließlich die Aldoladdition und -kondensation, weil deren Geschwindigkeit höher ist als die der Cannizzaro-Reaktion. Bei besonderen Reaktionsbedingungen können jedoch auch aldolisierbare Aldehyde bestimmter Struktur mit einem Wasserstoff-Atom in α-Stellung der Cannizzarro-Reaktion unterliegen.

So ist aus US-A-3,398,166 bekannt, daß C₄₋₁₆-Aldehyde mit nur einem Wasserstoff-Atom in α-Stellung in Gegenwart einer 30-50%igen wäßrigen Lösung eines Alkali- oder Erdalkalimetallhydroxids bei 40-250°C in den entsprechenden Alkohol und die zugehörige Carbonsäure disproportionieren. Im Fall von 2-Ethylhexanal werden in Gegenwart einer 50%igen Natronlauge, bezogen auf 100% Aldehyd, 48.5% 2-Ethylhexanol und 49% Natrium-ethylhexanoat gebildet.

Die Claisen-Tischtschenko-Reaktion tritt als weitere Nebenreaktion zur Aldolkondensation ein, wenn als Katalysator Aluminiumalkoholate verwendet werden, die zu schwach basisch sind, um die Aldolreaktion zu katalysieren. In diesem Fall unterliegen auch aldolisierbare aliphatische Aldehyde im Sinne einer Cannizzaro-Reaktion einer Umwandlung in das entsprechende Alkoholat und einen Ester der entsprechenden Carbonsäure.

Bekannt sind weitere Nebenreaktionen in Form von Folgereaktionen der Reaktionsprodukte der Aldolisierung. Hierbei handelt es sich beispielsweise um die Umsetzung des nach der Aldolkondensation vorliegenden α,β-ungesättigten Ketons mit weiteren Aldehydmolekülen zu höherkondensierten Verbindungen. In der US-A-3,291,82 1 wird hierzu beschrieben, daß aus C₄₋₁₀-Aldehyden mit nur einem Wasserstoff-Atom in α-Stellung bereits in Gegenwart einer lediglich 5-20%igen wäßrigen Lösung einer starken anorganischen Base bei 50-125°C durch Trimerisierung Glykolmonoester gebildet werden.

Zur Aldolkondensation von am β-C-Atom verzweigten Ketonen und am α-C-Atom verzweigten Aldehyden existiert nur wenig Literatur. Bekannt ist aus der GB-PS-446,026 die Aldolkondensation von Methylisobutylketon mit aliphatischen Aldehyden mit mindestens 8 C-Atomen unter Bildung von α,β-ungesättigten Ketonen. Insbesondere wird die Aldolkondensation von Methylisobutylketon mit 2-Ethylhexanal bei einer Temperatur von weniger als 25°C in Gegenwart einer Katalysatorlösung in Form methanolischer Kalilauge beschrieben. Alternativ zu Methanol als Lösungsmittel für den Katalysator können auch andere gegenüber den Reaktanden inerte, gleichzeitig aber flüchtige Substanzen, z.B. Ethanol, eingesetzt werden. Die beschriebene Verwendung von Alkalimetallhydroxiden als Katalysator hat in diesem Fall den Nachteil, daß infolge der Methanol-Anwesenheit eine größere Menge des Alkalimetalls mit in die bei der Reaktion gebildete organische Produktphase übergeht. Diese Produktphase muß anschließend zur Entfernung des Alkalimetalls entsprechend aufgearbeitet werden, z.B. durch aufwendiges Waschen mit Wasser, was wiederum zu großen Mengen an alkalibelastetem Abwasser führt.

Die Aufgabe der vorliegenden Erfindung bestand daher darin, ein verbessertes Verfahren zur Herstellung von gesättigten Alkoholen durch Aldolkondensation von am β-C-Atom verzweigten Ketonen und am α-C-Atom verzweigten Aldehyden sowie eine nachfolgende Hydrierung zur Verfügung zu stellen.

Gelöst wird diese Aufgabe durch ein Verfahren zur Herstellung von gesättigten Alkoholen umfassend eine Aldolkondensation von am β-C-Atom verzweigten Alkylmethylketonen mit 6 - 8 Kohlenstoffatomen und am α-C-Atom verzweigten Aldehyden mit 4 - 15 Kohlenstoffatomen unter Bildung von α,β-ungesättigten Ketonen und eine nachfolgende Hydrierung der α,β-ungesättigten Ketone zu den ensprechenden gesättigten Alkoholen, dadurch gekennzeichnet, daß die Aldolkondensation in Gegenwart einer 30-55 %igen, wäßrigen Lösung eines Alkalimetallhydroxids bei einer Temperatur von 60-130°C durchgeführt wird.

Vorzugsweise erfolgt die Aldolkondensation in Gegenwart einer 40-50 %igen wäßrigen Lösung eines Alkalimetallhydroxids. Weiterhin ist es zweckmäßig, die Aldolkondensation bei einer Temperatur von 80-120°C durchzuführen. Umsatz und Selektivität des erfindungsgemäßen Verfahrens sind exzellent. Nebenreaktionen der Keton- und Aldehyd-Edukte z.B. in Form der Cannizzaro- oder Claisen-Tischtschenko-Reaktion treten nur in ganz minimalem Ausmaß auf. So ist z.B. der bei der Cannizzaro-Reaktion aus dem Aldehyd entstehende Alkohol sowie die entsprechende Carbonsäure nur in minimalen Mengen von 0,1 - 0,6 % im Reaktionsgemisch zu finden.

Dies ist ein überraschendes Ergebnis, da die gewählten Reaktionsbedingungen der Aldolkondensation, d.h. die Reaktionstemperatur und die hohe Konzentration der als Katalysator eingesetzten wäßrigen Lösung von Alkalimetallhydroxiden, diese Nebenreaktionen im Sinne der Offenbarung von US-A-3,398,166 sowie US-A-3,291, 821 stark begünstigen sollten.

Im erfindungsgemäßen Verfahren stellt sich in der Aldolkondensation ein Gleichgewicht zwischen den α,β-ungesättigten Ketonen und den nicht umgesetzten Ausgangsverbindungen ein. Verwendet man beispielsweise 0,5 mol einer 40%igen wäßrigen Natronlauge, bezogen auf 1,05 mol Methylisobutylketon und 1,0 mol 2-Ethylhexanal, so liegt der Anteil des α,β-ungesättigten Ketons nach einer Reaktionszeit von 3 Stunden im Gleichgewicht bei etwa 80%.

Die Reaktionszeit, die prinzipiell für die Einstellung des Gleichgewichts zwischen den α,β-ungesättigten Ketonen und den nicht umgesetzten Ausgangsverbindungen benötigt wird, hängt von den gewählten Reaktionsbedingungen ab. Das erfindungsgemäße Verfahren kann insbesondere so durchgeführt werden, daß das bei der Aldolkondensation durch Dehydratisierung der β-Hydroxycarbonylverbindung gebildete Wasser entweder während der Reaktion laufend aus dem Reaktionsgemisch abgetrennt oder aber nicht abgetrennt wird. Wird das Wasser z.B. über einen Wasserabscheider entfernt, so liegt die Reaktionszeit bis zur Gleichgewichtseinstellung bei Anwendung einer 40%igen wäßrigen Lösung des Alkalimetallhydroxids bei ca. 60 min. Bei Nichtentfernung des Wassers, ansonsten aber identischen Bedingungen, erfolgt die Gleichgewichtseinstellung erst nach 180-240 min, da durch das gebildete Wasser die Konzentration der eingesetzten Alkalimetallhydroxid-Lösung allmählich abnimmt.

So sinkt beispielsweise die Konzentration einer 50 %igen Natronlauge, von der 0,5 mol eingesetzt werden, bis zum quantitativen Umsatz von 1 mol Aldehyd auf 34%. Bei Einsatz einer 40%igen Natronlauge wird unter analogen Bedingungen ein Absinken der Konzentration auf 29% beobachtet.

Bevorzugt ist daher die Ausführungsform des erfindungsgemäßen Verfahrens unter Entfernung des Reaktionswassers, bei der somit die ursprünglich eingesetzte Konzentration der Alkalimetallhydroxid-Lösung über die gesamte Reaktionszeit konstant gehalten wird.

Diese Verfahrensvariante hat zusätzlich den Vorteil, daß die den Katalysator enthaltende wäßrige Phase bei Reaktionsende, d.h. Erreichung des Gleichgewichtszustandes, nach Abtrennung von der organischen Produktphase ohne weitere Behandlung mehrfach recycliert werden kann.

Im erfindungsgemäßen Verfahren werden am β-C-Atom verzweigte Alkylmethylketone mit 6 - 8 Kohlenstoffatomen, bevorzugt Methylisobutylketon, eingesetzt, sowie am α-C-Atom verzweigte Aldehyde mit 4 - 15, bevorzugt 5-12 Kohlenstoffatomen, insbesondere 2-Ethylhexanal.

Das am β-C-Atom verzweigte Keton, der am α-C-Atom verzweigte Aldehyd sowie das in wäßriger Lösung vorliegende Alkalimetallhydroxid werden in einem molaren Verhältnis von (0.90-1.1) :1: (0.15-1), insbesondere (0.95-1) :1: (0.35-0.75) eingesetzt. Größere Ketonüberschüsse liefern keine Selektivitätsverbesserungen, und höhere Aldehydmengen ergeben keinen Umsatzvorteil.

Das erfindungsgemäße Verfahren kann kontinuierlich oder diskontinuierlich durchgeführt werden, bevorzugt.ist die kontinuierliche Fahrweise.

Die Hydrierung wird üblicherweise in heterogener Phase an Raney-Nickel oder Metall Träger Katalysatoren, bevorzugt Nickel-Katalysatoren, bei Temperaturen von 80-180°C, insbesondere 100-140°C und Drücken von 1-30 MPa, insbesondere 8-12 MPa durchgeführt. Hydrierungen unter diesen Bedingungen sind allgemein bekannt und beispielsweise in Methodicum Chimicum, Band 5, Hrsg. J. Falbe, 1975, Seite 47f, beschrieben.

Insbesondere eignet sich das erfindungsgemäße Verfahren zur Herstellung von 7-Ethyl-2-methyl-undecanol-4 (Tetradecanol) durch Aldclkondensation von Methylisobutylketon und 2-Ethylhexanal in Gegenwart einer 40-50%igen wäßrigen Natronlauge bei 90-110°C unter Bildung von 7-Ethyl-2-methyl-undecen-5-on-4 mit nachfolgender Hydrierung zum zuvor genannten Alkohol. Als Hydrierkatalysator wird hierbei insbesondere ein Kieselgur geträgerter Nickelkatalysator verwendet.

In Abhängigkeit von den für die Aldolkondensation gewählten Bedingungen und dem damit verbundenen Gehalt des α,β-ungesättigten Ketons im Reaktionsgemisch der Aldolkondensation sind im Hydriergemisch bis zu 85 % 7-Ethyl-2-methyl-undecanol-4 vorhanden.

### Beispiele:

### Beispiel 1

### Herstellung von 7-Ethyl-2-methyl-undecanol-4

Unter guter Durchmischung werden innerhalb von 100 Minuten aus getrennten Vorlagen 400,0 g 50%ige Natronlauge sowie eine Mischung aus 1062,0 g (10,6 mol) Methylisobutylketon und 1294,0 g (10,1 mol) 2-Ethylhexanal in einen 4 1-Rundkolben getropft. Das Gemisch wird anschließend auf 100°C erwärmt und 60 Minuten gerührt. Nach Beendigung der Reaktion läßt man abkühlen. Entsprechend der gaschromatographischen Analyse setzt sich die organische Phase (2215,0 g) wie folgt zusammen:

| | |
|---|---|
| 8,2 % | Methylisobutylketon |
| 4,0 % | 2-Ethylhexanal |
| 0,2 % | 2-Ethylhexanol |
| 78,9 % | 7-Ethyl-2-methyl-undecen-5-on-4 |
| 8,7 % | Sonstige. |

Bezogen auf das eingesetzte 2-Ethylhexanal beträgt die Ausbeute an 7-Ethyl-2-methyl-undecen-5-on-4 82,3% der Theorie.

Das Produkt kann ohne weitere Reinigungsoperationen direkt in die Hydrierung eingesetzt werden. Die Hydrierung wird in Gegenwart eines Ni-Katalysators ™CelActiv Ni 52/35 bei einer Temperatur von 120°C und einem Druck von 10 MPa durchgeführt. Dieser Ni-Katalysator ™CelActiv Ni 52/35 ist ein Handelsprodukt der Celanese GmbH. Das Hydrierprodukt weist folgende Zusammensetzung auf:

| | |
|---|---|
| 8.4 % | 2-Methyl-pentanol-4 |
| 4,0 % | 2-Ethylhexanol |
| 78,3 % | 7-Ethyl-2-methyl-undecanol-4 |
| 9,3 % | Sonstige. |

Die Reingewinnung des 7-Ethyl-2-methyl-undecanol-4 erfolgt durch abschließende Destillation bei 132°C und einem Druck von 10 mbar.

### Beispiel 2

### Herstellung von 7-n-Propyl-2-methyl-dodecanol-4

Unter guter Durchmischung werden innerhalb von 90 Minuten aus getrennten Vorlagen 62,5 g 40%ige Natronlauge sowie eine Mischung aus 265,5 g Methylisobutylketon (2,65 mol) und 390,0 g 2-n-Propylheptanal (2,50 mol) in einen 1 l-Rundkolben getropft. Das Gemisch wird anschließend auf 108 - 110°C erwärmt und 4 Stunden gerührt. Nach Beendigung der Reaktion läßt man abkühlen. Entsprechend der gaschromatographischen Analyse setzt sich die organische Phase (621,9 g) wie folgt zusammen:

| | |
|---|---|
| 11,0 % | Methylisobutylketon |
| 13,1 % | 2-n-Propylheptanal |
| 0,9 % | 2-n-Propylheptanol |
| 70,1 % | 7-n-Propyl-2-methyl-dodecen-5-on-4 |
| 4,9 | Sonstige. |

Bezogen auf das eingesetzte 2-Propylheptanal beträgt die Ausbeute an 7-n-Propyl-2-methyl-dodecen-5-on-4 73,3% der Theorie.

Das Produkt wird in Gegenwart eines Ni-Katalysators ™CelActiv Ni 52/35 bei einer Temperatur von 120°C und einem Druck von 10 MPa hydriert. Die Reingewinnung des 7-n-Propyl-2-methyl-dodecanol-4 erfolgt durch abschließende Destillation bei 170°C und einem Druck von 50 mbar.

### Beispiel 3

### Herstellung von 2,7-Dimethyl-nonanol-4

Unter guter Durchmischung werden innerhalb von 90 Minuten aus getrennten Vorlagen 62,5 g 40%ige Natronlauge sowie eine Mischung aus 265,5 g Methylisobutylketon (2,65 mol) und 215,0 g 2-Methylbutanal (2,50 mol) in einen 1 l-Rundkolben getropft. Das Gemisch wird anschließend auf 90°C erwärmt und 4 Stunden gerührt. Nach Beendigung der Reaktion läßt man abkühlen. Entsprechend der gaschromatographischen Analyse setzt sich die organische Phase (438,5 g) wie.folgt zusammen:

| | |
|---|---|
| 1,0 % | 2-Methylbutanal |
| 7,6 % | Methylisobutylketon |
| 0,5 % | 2-Methylbutanol |
| 73,2 % | 2,7-Dimethyl-nonen-5-on-4 |
| 18,2 % | Sonstige. |

Bezogen auf das eingesetzte 2-Methylbutanal beträgt die Ausbeute an 2,7-Dimethyl-nonen-5-on-4 76,4% der Theorie.

Das Produkt wird in Gegenwart eines Ni-Katalysators ™CelActiv Ni 52/35 bei einer Temperatur von 120°C und einem Druck von 10 MPa hydriert. Die Reingewinnung des 2,7-Dimethyl-nonanol-4 erfolgt durch abschließende Destillation bei 127°C und einem Druck von 50 mbar.

### Beispiel 4

### Herstellung von 2,7-Dimethyl-hexadecanol-4

Unter guter Durchmischung werden innerhalb von 90 Minuten aus getrennten Vorlagen 31,3 g 40%ige Natronlauge sowie eine Mischung aus 132,8 g Methylisobutylketon (1,33 mol) und 230,4 g 2-Methylundecanal (1,25 mol) in einen 1 l-Rundkolben getropft. Das Gemisch wird anschließend auf 112 -118°C erwärmt und 4 Stunden gerührt. Nach Beendigung der Reaktion läßt man abkühlen. Entsprechend der gaschromatographischen Analyse setzt sich die organische Phase (621,9 g) wie folgt zusammen:

| | |
|---|---|
| 10,8 % | Methylisobutylketon |
| 8,4 % | 2-Methylundecanal |
| 0,9 % | 2-Methylundecanol |
| 77,6 % | 2,7-Dimethyl-hexadecen-5-on-4 |
| 3,2 % | Sonstige. |

Bezogen auf das eingesetzte 2-Methylundecanal beträgt die Ausbeute an 2,7-Dimethyl-hexadecen-5-on-4 78,0% der Theorie.

Das Produkt wird in Gegenwart eines Ni-Katalysators ™CelActiv Ni 52/35 bei einer Temperatur von 120°C und einem Druck.von 10 MPa hydriert. Die Reingewinnung des 2,7-Dimethyl-hexadecanol-4 erfolgt durch abschließende Destillation bei 221°C und einem Druck von 50 mbar.

### Beispiele 5 - 17

### Diskontinuierliche Aldolkondensation von Methylisobutylketon und 2-Ethylhexanal zu 7-Ethyl-2-methyl-undecen-5-on-4 (I) ohne Wasserabtrennung

Die Aldolkondensationen der Beispiele 5-17 werden nach der folgenden Vorschrift durchgeführt:

Das Gemisch aus Methylisobutylketon und 2-Ethylhexanal sowie die Natronlauge werden innerhalb von 10 Minuten in einen 500 ml Rundkolben eingetropft. Durch die sofort einsetzende exotherme Reaktion findet eine Erwärmung auf 40-50°C statt. Nach dem Zutropfende wird die gewünschte Reaktionstemperatur durch zusätzliche Wärmezufuhr innerhalb von 10-25 Minuten erreicht. Startpunkt für die Bestimmung der Reaktionszeit ist das Erreichen der Reaktionstemperatur, die Aufheizphase bleibt somit unberücksichtigt.

Die Konzentration der wässrigen Natronlauge, die Menge der wässrigen Natronlauge, bezogen auf den Aldehyd, Reaktionszeit sowie Temperatur sind in den nachfolgenden Tabellen aufgeführt.

**Tabelle 1**

| Einfluß der Katalysatormenge * | | | | |
|---|---|---|---|---|
| Beispiel | 5 | 6 | 7 | 8 |
| Reaktionszeit (min.) | 240 | 240 | 240 | 120 |
| Molares Verhältnis Keton:Aldehyd:NaOH (1.05:1:X) X | 0.25 | 0.375 | 0.5 | 0.75 |
| Anteil I im Rohprodukt | 64.9 | 72.5 | 74.8 | 73.8 |

| | | | | |
|---|---|---|---|---|
| * eingesetzt wird eine 50 %ige Natronlauge und eine Reaktionstemperatur von 80°C. | | | | |

**Tabelle 2**

| Einfluß der Katalysatorkonzentration * | | | |
|---|---|---|---|
| Beispiel | 9 | 10 | 11 |
| Reaktionszeit (min.) | 240 | 240 | 240 |
| Konzentration NaOH (%) | 30 | 40 | 50 |
| Anteil I im Rohprodukt | 68.1 | 78.3 | 79.4 |

| | | | |
|---|---|---|---|
| * es wird mit einem molaren Verhältnis von Keton: Aldehyd: NaOH von 1,05 : 1 : 0,5 und einer Temperatur von 100°C gearbeitet. | | | |

**Tabelle 3**

| Einfluß der Reaktionstemperatur * | | | | | | |
|---|---|---|---|---|---|---|
| Beispiel | 12 | 13 | 14 | 15 | 16 | 17 |
| Reaktionszeit (min.) | 240 | 240 | 240 | 120 | 60 | 60 |
| Reaktionstemperatur (°C) | 60 | 80 | 90 | 100 | 110 | 120 |
| Anteil I im Rohprodukt | 71.3 | 74.8 | 79.1 | 79.5 | 77.2 | 72.5 |

| | | | | | | |
|---|---|---|---|---|---|---|
| * es wird mit einem molaren Verhältnis von Keton: Aldehyd : NaOH von 1,05 : 1 : 0,5 und einer Temperatur von 100°C gearbeitet. | | | | | | |

### Beispiele 18-24

### Diskontinuierliche Aldolkondensation von Methylisobutylketon und 2-Ethylhexanal zu 7-Ethyl-2-methyl-undecen-5-on-4 (I) mit Abtrennung des Reaktionswassers

Die Versuchsdurchführung erfolgt in den Beispielen 18-24 zunächst analog zu den Beispielen 5-17. Nach Ende des Zutropfens der wässrigen Natronlauge und des Gemischs aus Methylisobutylketon und 2-Ethylhexanal sowie dem Aufheizen auf Reaktionstemperatur wird der Druck in der Apparatur bis zum Erreichen des Siedepunkts verringert und im weiteren Reaktionsverlauf so eingestellt, daß die gewünschte Reaktionstemperatur gehalten wird. Über einen Wasserabscheider wird nun die jeweils theoretisch zu erwartende Menge an Reaktionswasser selektiv abdestilliert, so daß die zu Beginn eingesetzte Laugenkonzentration im Reaktionsverlauf weitgehend konstant bleibt.

Die in den Beispielen 18-23 eingesetzte Konzentration der wässrigen Natronlauge, die Reaktionszeit sowie die Reaktionstemperatur ist in der nachfolgenden Tabelle 4 aufgeführt. In allen Beispielen wurde mit einem molaren Verhältnis Keton: Aldehyd: NaOH von 1.05 : 1 : 0.5 gearbeitet.

**Tabelle 4**

| Einfluß der Reaktionstemperatur bei Verwendung verschieden konzentrierter Natronlauge | | | | | | |
|---|---|---|---|---|---|---|
| Beispiel | 18 | 19 | 20 | 21 | 22 | 23 |
| Konzentration der NaOH | 30 | 30 | 40 | 40 | 50 | 50 |
| Reaktionszeit (min.) | 135 | 135 | 75 | 75 | 135 | 15 |
| Reaktionstemperatur (°C) | 90 | 100 | 90 | 100 | 90 | 100 |
| Anteil I im Rohprodukt | 42.7 | 74.2 | 73.0 | 80.6 | 78.9 | 79.1 |

Wie aus Tabelle 4 hervorgeht, reduziert sich die zur Gleichgewichtseinstellung notwendige Reaktionszeit gegenüber den Versuchen ohne Wasserabtrennung von ca. 3h auf 1h.

Durch das folgende Beispiel 24 wird ein weiterer Vorteil dieser Ausführungsform des erfindungsgemäßen Verfahrens dargestellt. Gearbeitet wird mit einem molaren Verhältnis Methylisobutylketon:2-Ethylhexanal:NaOH von 1.05 : l : 0.5 und einer 40%igen Natronlauge bei einer Reaktionstemperatur von 100°C, einem Druck von 500 mbar und einer Reaktionszeit von 60 Minuten, wobei die Katalysatorphase fünfmal erneut eingesetzt wird. Der im jeweiligen Produktgemisch enthaltene Anteil an 7-Ethyl-2-methylundecen-5-on-4 ist in Tabelle 5 aufgeführt.

Es zeigt sich, daß mit zunehmender Zahl der Recyclierungen der Katalysatorphase bei einer geringfügig steigenden Selektivität der Gehalt an 7-Ethyl-2-methyl-undecen-5-on-4 von 81,1 % lediglich um 2,8% auf 78,3% (nach dem 4. Wiedereinsatz) fällt.

**Tabelle 5**

| Beispiel 24 | | Wiedereinsatz | | | |
|---|---|---|---|---|---|
| | Nach der 1. Aldolkondensation | 1 | 2 | 3 | 4 |
| Anteil an 7-Ethyl-2-methyl-undecen-5-on-4 | 81,12 | 80,75 | 79,25 | 77,76 | 78,34 |

## Patentansprüche

1. Verfahren zur Herstellung von gesättigten Alkoholen umfassend
1) eine Aldolkondensation von am β-C-Atom verzweigten Alkylmethylketonen mit 6 - 8 Kohlenstoffatomen mit am α-C-Atom verzweigten Aldehyden mit 4 - 15 Kohlenstoffatomen unter Bildung von α,β-ungesättigten Ketonen und
2) eine nachfolgende Hydrierung der α,β-ungesättigten Ketone zu gesättigten Alkoholen,
**dadurch gekennzeichnet, daß** die Aldolkondensation in Gegenwart einer 30-55%igen wäßrigen Lösung eines Alkalimetallhydroxids bei einer Temperatur von 60-130°C durchgeführt wird.

2. Verfahren nach Anspruch 1, **dadurch gekennzeichnet, daß** die Aldolkondensation in Gegenwart einer 40-50 %igen, wäßrigen Lösung eines Alkalimetallhydroxids durchgeführt wird.

3. Verfahren nach Anspruch 1 oder 2, **dadurch gekennzeichnet, daß** die Aldolkondensation bei einer Temperatur von 80-120°C durchgeführt wird.

4. Verfahren nach einem oder mehreren der Ansprüche 1-3, **dadurch gekennzeichnet, daß** das bei der Aldolkondensation in Schritt 1 entstehende Wasser während der Reaktion laufend aus dem Reaktiongemisch abgetrennt wird .

5. Verfahren nach einem oder mehreren der Ansprüche 1-4, **dadurch gekennzeichnet, daß** Methylisobutylketon sowie am α-C-Atom verzweigte Aldehyde mit 5-12 Kohlenstoffatomen eingesetzt werden.

6. Verfahren nach Anspruch 5, **dadurch gekennzeichnet, daß** als am α-C-Atom verzweigter Aldehyd 2-Ethylhexanal eingesetzt wird.

7. Verfahren nach einem oder mehreren der Ansprüche 1-6, **dadurch gekennzeichnet, daß** das am β-C-Atom verzweigte Keton, der am α-C-Atom verzweigte Aldehyd sowie das in wäßriger Lösung vorliegende Alkalimetallhydroxid in einem molaren Verhältnis von (0.90-1.1) :1: (0.15-1) eingesetzt werden.

8. Verfahren nach Anspruch 7, **dadurch gekennzeichnet, daß** das am β-C-Atom verzweigte Keton, der am α-C-Atom verzweigte Aldehyd sowie das in wäßriger Lösung vorliegende Alkalimetallhydroxid in einem molaren Verhältnis von (0.95-1) :1: (0.35-0.75) eingesetzt werden.

## Claims

1. A process for preparing saturated alcohols comprising
1) an aldol condensation of alkyl methyl ketones which have 6-8 carbon atoms and are branched at the β-carbon atom with aldehydes which have 4-15 carbon atoms and are branched at the α-carbon atom to form α,β-unsaturated ketones and
2) subsequent hydrogenation of the α,β-unsaturated ketones to give saturated alcohols,
wherein the aldol condensation is carried out at a temperature of 60-130°C in the presence of a 30-55% strength aqueous solution of an alkali metal hydroxide.

2. The process as claimed in claim 1, wherein the aldol condensation is carried out in the presence of a 40-50% strength aqueous solution of an alkali metal hydroxide.

3. The process as claimed in claim 1 or 2, wherein the aldol condensation is carried out at a temperature of 80-120°C.

4. The process as claimed in one or more of claims 1 to 3, wherein the water formed in the aldol condensation in step 1 is continually separated off from the reaction mixture during the reaction.

5. The process as claimed in one or more of claims 1 to 4, wherein methyl isobutyl ketone plus aldehydes which have 5-12 carbon atoms and are branched at the α-carbon atom are used.

6. The process as claimed in claim 5, wherein the aldehyde which is branched at the α-carbon atom is 2-ethylhexanal.

7. The process as claimed in one or more of claims 1 to 6, wherein the ketone which is branched at the β-carbon atom, the aldehyde which is branched at the α-carbon atom and the alkali metal hydroxide present in aqueous solution are used in a molar ratio of (0.90-1.1) : 1 : (0.15-1).

8. The process as claimed in claim 7, wherein the ketone which is branched at the β-carbon atom, the aldehyde which is branched at the α-carbon atom and the alkali metal hydroxide present in aqueous solution are used in a molar ratio of (0.95-1) : 1 : (0.35-0.75).

## Revendications

1. Procédé pour la préparation d'alcools saturés comprenant
1) une condensation d'aldol d'alkylméthylcétones ramifiées sur l'atome β-C avec 6-8 atomes de carbone avec des aldéhydes ramifiés sur l'atome α-C avec 4-15 atomes de carbone avec la formation de cétones α,β-insaturées et
2) une hydrogénation subséquente des cétones α,β-insaturées en alcools saturés,
**caractérisé en ce que** la condensation d'aldol est réalisée en présence d'une solution aqueuse d'un hydroxyde de métal alcalin à 30-55 % à une température de 60-130°C.

2. Procédé selon la revendication 1, **caractérisé en ce que** la condensation d'aldol est réalisée en présence d'une solution aqueuse d'un hydroxyde de métal alcalin à 40-50 %.

3. Procédé selon la revendication 1 ou 2, **caractérisé en ce que** la condensation d'aldol est réalisée à une température de 80-120°C.

4. Procédé selon l'une ou plusieurs quelconques des revendications 1-3, **caractérisé en ce que** l'eau produite dans la condensation d'aldol de l'étape 1 est éliminée du mélange réactionnel au cours de la réaction.

5. Procédé selon l'une ou plusieurs quelconques des revendications 1-4, **caractérisé en ce que** sont utilisés de la méthylisobutylcétone ainsi que des aldéhydes ramifiés sur l'atome α-C avec 5-12 atomes de carbone sont utilisés.

6. Procédé selon la revendication 5, **caractérisé en ce que** l'on utilise comme aldéhyde ramifié sur l'atome α-C du 2-éthylhexanal.

7. Procédé selon l'une ou plusieurs quelconques des revendications 1-6, **caractérisé en ce que** la cétone ramifiée sur l'atome β-C, l'aldéhyde ramifié sur l'atome α-C ainsi que l'hydroxyde de métal alcalin présent dans la solution aqueuse sont utilisés avec un rapport molaire de (0,90-1,1):1:(0,15-1).

8. Procédé selon la revendication 7, **caractérisé en ce que** la cétone ramifiée sur l'atome β-C, l'aldéhyde ramifié sur l'atome a-C ainsi que l'hydroxyde de métal alcalin présent dans la solution aqueuse sont utilisés avec un rapport molaire de (0,95-1):1:(0,35-0,75).
